**Europäisches Patentamt**

**European Patent Office**

**Office européen des brevets**

(11) Publication number: **0 142 161**
**B1**

# EUROPEAN PATENT SPECIFICATION

(45) Date of publication of patent specification: **23.01.91**

(51) Int. Cl.⁵: **A 61 B 5/0428,** A 61 N 1/08

(21) Application number: **84113682.3**

(22) Date of filing: **13.11.84**

(54) **Apparatus for processing EKG signals.**

(30) Priority: **14.11.83 US 551628**

(43) Date of publication of application:
**22.05.85 Bulletin 85/21**

(45) Publication of the grant of the patent:
**23.01.91 Bulletin 91/04**

(84) Designated Contracting States:
**DE FR GB**

(56) References cited:
**DE-B-2 805 681**
**US-A-3 897 774**
**US-A-3 986 496**
**US-A-4 226 245**

(73) Proprietor: **Hewlett-Packard Company**
**Mail Stop 20 B-O, 3000 Hanover Street**
**Palo Alto, California 94304 (US)**

(72) Inventor: **Regan, Richard J.**
**20 Columbus Avenue**
**Beverly Massachusetts 01915 (US)**

(74) Representative: **Liesegang, Roland, Dr.-Ing.**
**FORRESTER & BOEHMERT Widenmayerstrasse**
**4 Postfach 22 01 37**
**D-8000 München 22 (DE)**

Courier Press, Leamington Spa, England.

**Description**

The invention relates to an apparatus for processing EKG signals having pace pulses therein according to the preamble of claim 1 (US—A—3,897,774).

When a patient has a pacemaker, the electrical pulses it provides are added to the EKG waves derived from that patient by the electrodes of an electrocardiograph. Depending on the pacemaker employed and its adjustment the width of the pacemaker pulses in the electrocardiogram may range from about 100 to about 2000 microseconds, and their amplitude at most electrode locations usually exceeds that of the EKG wave by a substantial amount. A conventional electrocardiograph may be disturbed by the high amplitudes of the pace pulse so as to display it with an incorrect amplitude.

Pacemakers in which the pace pulses are capacitively coupled to the patient cause a voltage of the opposite polarity following the pace pulse. This voltage, hereinafter referred to as "after-voltage" can last for as long as several milliseconds following the pace pulse, thus distorting the electrocardiogram.

A further problem arises when an electrocardiograph is used which displays a plurality of EKG waves at the same time derived from different electrodes coupled to a patient's body by rapidly sampling each wave in sequence and using the samples derived from each wave to reproduce it.

The pace pulses in some of the EKG waves may be weak or even nonexistent, but it is desirable that they be appropriately displayed in each wave. Furthermore in these sampling electrocardiographs the electrocardiogram is also impaired by the after-voltage.

US—A—3,897,774 discloses a signal processing circuit for use in a digital memory-type EKG display system, wherein analogue EKG-signals are periodically sampled and converted into digital signals for processing and wherein a display comprises an amplitude hold circuit which maintains the instantaneous amplitude of pacemaker pulses contained in the analogue EKG signal constant for a sufficient period of time to ensure recognition of the pulses in the next sampling. Thus, substitute pulses are generated. From these substitute pulses, however, no information about the character of the original pace pulses can be derived.

The object of the invention is to provide an apparatus of the kind mentioned in the first part of claim 1, wherein the effects of the after-voltage on the electrocardiogram are avoided and wherein the processing circuit is not disturbed by pace pulses having higher amplitudes than the EKG signals. Particularly such apparatus should be capable to generate a substitute pulse, the amplitude of which gives information about the character of the pace pulse.

According to the invention this object is solved by the features of the characterizing portion of claim 1.

By opening the switch any after-voltage that may be present is prevented from reaching the output, so that a distortion of the electrocardiogram is avoided.

Alternative solutions of the mentioned object are claimed in claims 3 and 4.

A preferred embodiment of the apparatus according to claim 1 is claimed in claim 2.

The invention provides the advantage that it can be utilized with any type of pacemaker or pace pulse.

The invention provides the further advantage that its use in a sampling electrocardiograph, which displays a plurality of EKG waves, ensures identical amplitudes of the pace pulse in each EKG wave.

Subsequently, various embodiments of the invention are explained in detail with reference to the drawings. The drawings show:

Figure 1 illustrates an electrocardiographic system employing sampling so that four EKG signals may be displayed at the same time.

Figure 2a is an electrocardiogram produced by a system of Figure 1 that uses coupling circuits of the prior art.

Figure 2b is an electrocardiogram produced by a system of Figure 1 that uses the coupling circuits of the invention.

Figure 3a illustrates a pace pulse and the after voltage that follows it when it is capacity-coupled.

Figure 3b illustrates the effect of filtering and sampling a wave such as shown in Figure 3a.

Figures 4 and 5 are block diagrams of EKG processing circuits in which the voltage produced in response to pace pulses is proportional to their amplitude.

Figure 4G is a schematic diagram of a circuit for Figure 4.

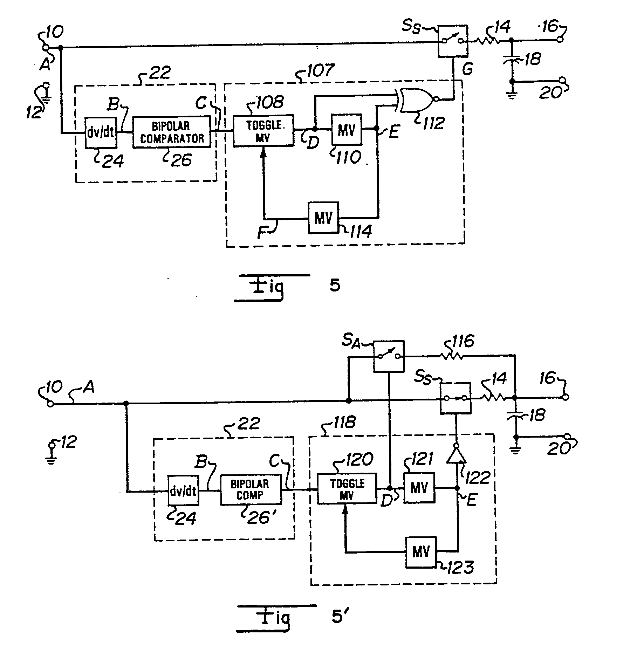

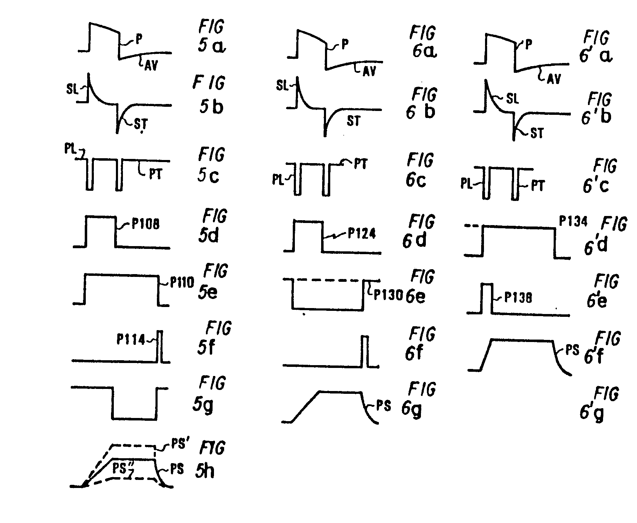

The circuits shown in Figs. 4, 4G and 5 do not exemplify the invention, since they do not include an auxiliary switch.

Figures 4a, 4b and 4c are waveforms appearing at like-lettered points in Figures 4 and 4'.

Figures 4d, 4e and 4f are waveforms appearing at like-lettered points in Figure 4.

Figure 4' is a block diagram of a second embodiment of the preferred specie of an EKG processing circuit of this invention in which the amplitude of the substitute pulse $P_S$ is different from that in Figure 4.

Figures 4d', 4e' and 4f' are waveforms appearing at like-lettered points in Figure 4'.

Figures 5a, 5b, 5c, 5d, 5e, 5f, 5g and 5h are waveforms appearing at like-lettered points in Figures 5 and 5'.

Figure 5' is a block diagram of a second embodiment of the second specie of an EKG processing circuit of this invention in which the amplitude of the substitute pulse $P_S$ is different from that in Figure 5.

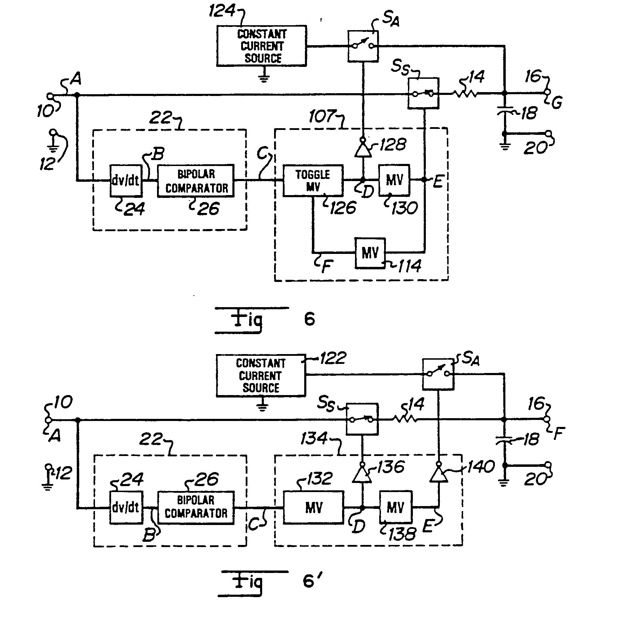

Figure 6 is a block diagram of a first embodiment of a third specie of an EKG processing circuit of this invention in which the substitute pulse $P_S$ is generated by a constant current source and the resulting voltage is related to the pace pulse width.

Figures 6a, 6b, 6c, 6d, 6e, 6f and 6g are waveforms appearing at like-lettered points in Figure 6.

Figure 6' is a second embodiment of the third specie of an EKG processing circuit of this invention in which the substitute pulse $P_S$ has a constant amplitude.

Figures 6'A through 6'G are waves that appear at like-lettered points in Figure 6'.

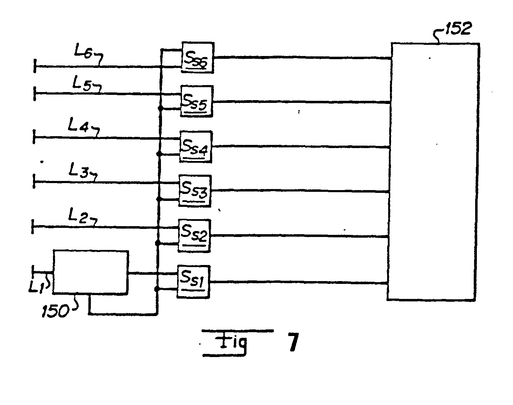

Figure 7 shows the use of a coupling circuit of this invention in one lead to control the signal switches in its own lead and in a plurality of other leads.

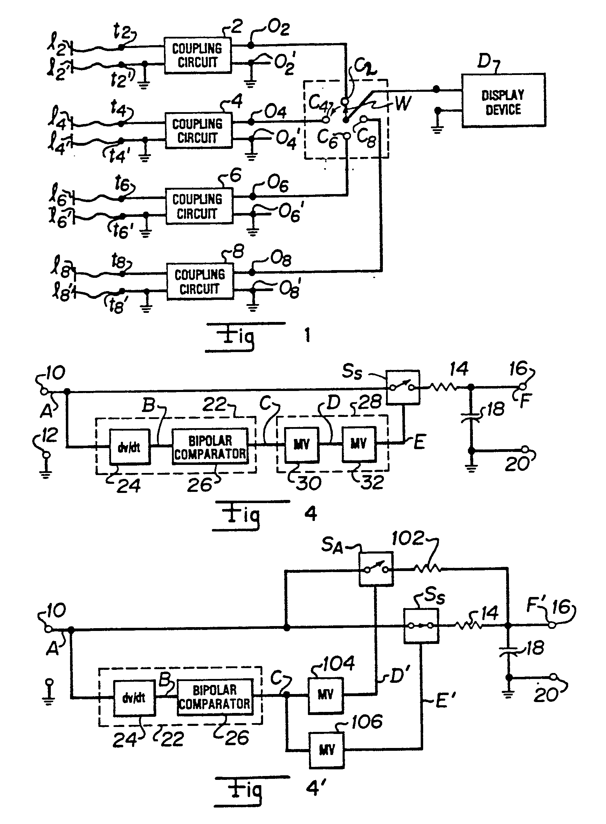

The electrocardiographic system schematically illustrated in Figure 1 has four sets of electrodes $e_2$, $e_2'$; $e_4$, $e_4'$; $e_6$, $e_6'$ and $e_8$, $e_8'$ that are respectively connected to the input terminals $t_2$, $t_2'$; $t_4$, $t_4'$; $t_6$, $t_6'$ and $t_8$, $t_8'$ of coupling circuits 2, 4, 6 and 8. The input terminals $t_2'$, $t_4'$, $t_6'$ and $t_8'$ are connected to a point of reference potential which may be floating ground. The output terminals of the coupling circuits 2, 4, 6 and 8 are $o_2$, $o_2'$; $o_4$, $o_4'$; $o_6$, $o_6'$ and $o_8$, $o_8'$ respectively, and output terminals $o_2'$, $o_4'$, $o_6'$ and $o_8'$ are connected to points of the reference potential just mentioned. The output terminals $o_2$, $o_4$, $o_6$ and $o_8$ are respectively connected to the contacts $c_2$, $c_4$, $c_6$ and $c_8$ of an electronic sampling switch S having a commutating contact W which is connected to the input of a display means D that respectively produces separate waves with the samples from the contacts $c_2$, $c_4$, $c_6$ and $c_8$ in a manner known to those skilled in the art.

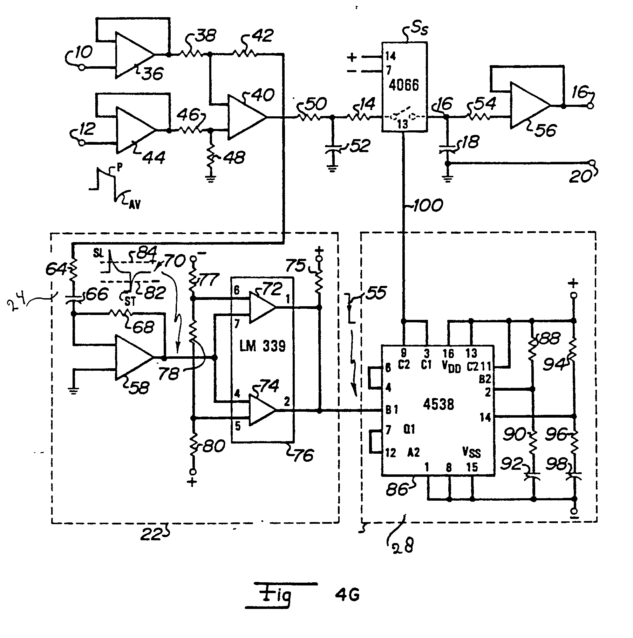

With the coupling circuits 2, 4, 6 and 8 of Figure 1 constructed in accordance with the prior art so as to have 100 Hz low pass filters, EKG waves produced by the display means D appeared as illustrated in Figure 2a, in which the R waves $R_2$ through $R_6$ have positive pace pulses $P_2$ through $P_6$ respectively occurring prior to them that vary in amplitude and, as noted in connection with the R waves $R_1$ through $R_7$, the pace pulses can even be entirely missing. The reason for this can be understood from the following. If a rectangular pace pulse is AC-coupled to the patient's body it will appear as a pulse P, illustrated in Figure 3a, followed by an after-voltage AV of the opposite polarity. The pulse P is the only effective part of the wave of Figure 3a and will be referred to referred to herein as a pace pulse. After passing through a 100-cycle low pass filter in the usual coupling circuit of Figure 1 the pace pulse P and its after-voltage AV appear as respectively indicated at P' and AV' of Figure 3b. Now if it is sampled at points $s_1$ through $s_8$ and applied to a sample-and-hold circuit, it will appear as indicated by the stepped wave O of Figure 3b. The representation of the pace pulse P' in the wave O has a smaller amplitude than if a sample occurred at the peak of the filtered pulse P'; and if no sample occurs within the pace pulse P' its representation will be zero as in connection with R waves $R_1$ and $R_7$ of Figure 2a. In a system using sampling, this phenomenon will happen regardless of whether the pacemaker is directly coupled or capacitively coupled.

It is also to be noted that the EKG waveforms of Figure 2a produced by the prior art contain negative pulses $AV_1$ through $AV_7$ that extend in a negative direction. They are caused by the negative after-voltage AV, such as shown in Figure 3a, which occurs when the pacemaker is capacitively coupled. Because of the duration of the after-voltage AV, a plurality of samples $s_3$ through $s_8$ occur during its filtered form AV' shown in Figure 3b. They appear as being widely separated in Figure 3b due to its expanded time scale but appear as a single pulse such as any one of $AV_1$ through $AV_7$ in the much-reduced time scale of the display. The amplitude of the negative pulses $AV_1$ through $AV_7$ is more nearly uniform than that of the pace pulses $P_1$ through $P_7$ because the after-voltage AV' is so broad that at least one of the samples occurs near its peak.

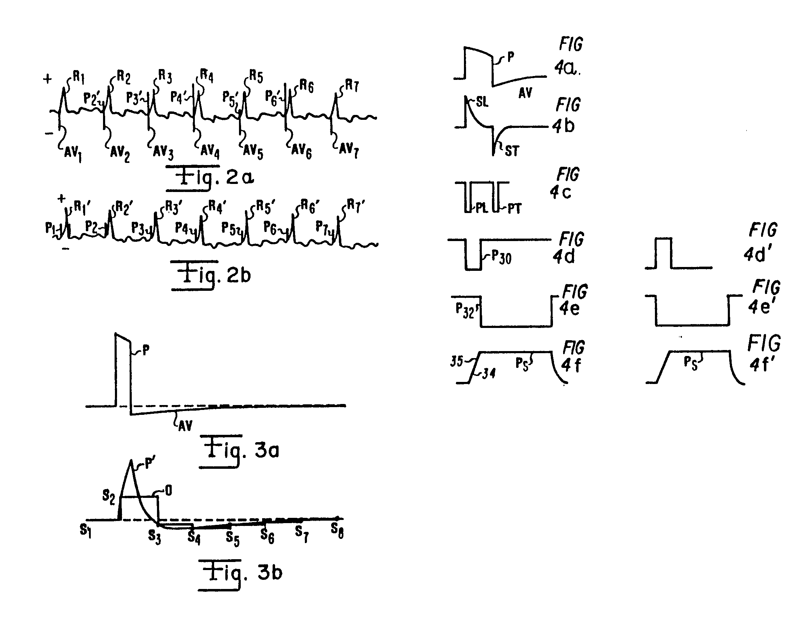

The EKG waves that were applied to the coupling circuits of Figure 1 in producing the display of Figure 2a were recorded and then applied to a processing circuit constructed in accordance with this invention. The display then appeared as in Figure 2b. Note that all pace pulses are represented, that all have the same amplitude and that no negative pulses such as $AV_1$ through $AV_7$ are present.

A block diagram of a first embodiment of a preferred specie of this invention that was actually used in obtaining the processed EKG signals of Figure 2b is shown in Figure 4. The voltage waves of Figures 4a through 4f that are used in explaining its operation appear at correspondingly lettered points. This circuit derives the substitute pulse $P_S$ by integrating a portion of a pace pulse P so that the substitute pulse $P_S$ has an amplitude proportional to the amplitude of a pace pulse.

The EKG signals to be processed are applied across input terminals 10 and 12, the latter being connected to a point of reference potential such as floating ground. A normally closed signal switch Ss and a resistor 14 are connected in series in either order between the input terminal 10 and an output terminal 16, and a capacitor 18 is DC-coupled between the output terminal 16 and an output terminal 20 that is connected to the point of reference potential. As in the prior art, the values of the resistor 14 and the capacitor 18 are such as to form a low pass filter having a cut-off just above the highest frequency of interest in the EKG signals, e.g., 100 cycles, so as to reject high frequency noise.

When a positive pace pulse P of Figure 4a arrives at the input 10, 12, its presence is detected by a pace pulse sensing means 22 coupled thereto that is comprised of a differentiation circuit 24 and a bipolar comparator 26. As shown in Figure 4b, the differentiation circuit 24 outputs a positive signal $S_L$ coincident with the leading edge of the pace pulse P and a negative signal $S_T$ coincident with the trailing edge of the pace pulse P. The bipolar comparator 26 produces pulses $P_L$

and $P_T$ of Figure 4c that are respectively coincident with the leading and trailing edges of the pace pulse P and are negative regardless of its polarity.

A signal switch control means 28 is herein shown as being comprised of a 50-microsecond monostable multivibrator 30 that is triggered by the negative-going edge of the pulse $P_L$ so as to produce a negative pulse $P_{30}$ (Figure 4d) and a 10-millisecond monostable multivibrator 32 that is triggered by the negative-going edge of a pulse $P_{30}$ so as to produce at its output a pulse $P_{32}$ (Figure 4e). When $P_{32}$ is low, it keeps the normally closed switch $S_s$ open so that the period during which the signal switch $S_s$ is made nonconductive starts during the pace pulse P.

Thus, the pace pulse P is integrated by the resistor 14 and the capacitor 18 only during the pulse $P_{30}$ of Figure 4d, which was 50 microseconds in the tests producing the wave of Figure 2b, so as to produce an increasing voltage across the capacitor 18 as indicated by the sloping line 34 Figure 4f. At the end of the pulse $P_{30}$ of Figure 4f, the integrated voltage across the capacitor 18 reaches a maximum value indicated at 35 that is proportional to the amplitude of the pace pulse P. The maximum value lasts as long as the switch $S_s$ is open, i.e., for a period equal to the duration of the pulse $P_{32}$. At the end of the pulse $P_{32}$, the switch $S_s$ closes and the voltage across the capacitor 18 assumes the value of the voltage at the input 10, 12. The actions just described form the pulse $P_S$ that is substituted for the pace pulse P.

The duration of the pulse $P_{32}$ and therefore the time after the pace pulse P during which the switch $S_s$ is nonconductive is such that the after-voltage AV will have a negligible effect on the integrated voltage produced across the capacitor 18. This depends, of course, on the duration and amplitude of the after-voltage AV, which can vary, but with present pacemakers a period of approximately 10 milliseconds has been found to be satisfactory.

Reference is now made to Figure 4G which schematically illustrates one form of circuit that may be used to carry out the functions just described in connection with the block diagram of Figure 4. Components corresponding to those of Figure 4 are designated in the same way, and the designations inside the boxes indicate the particular terminals of the solid state devices.

The input terminal 10 is coupled via a buffer amplifier 36 and a resistor 38 to one input of a differential amplifier 40, and a resistor 42 is connected between that input to the amplifier 40 and its output. The input terminal 12 is connected via a buffer amplifier 44 and a resistor 46 to the other input of the differential amplifier 40, and a resistor 48 is connected between that input of the amplifier 40 and a point of reference potential. A resistor 50 and a capacitor 52 that are connected in series from the output of the amplifier 40 to a point of reference potential form an additional low pass filter, not shown in Figure 4, that has no effect on the EKG signal but allows some latitude of adjustment of the narrow pace pulse to establish an exact scale factor or proportionality factor of the substitute pulse $P_S$ with respect to the actual pace pulse amplitude. The junction of the resistor 50 and the capacitor 52 is connected by the resistor 14 to the input of the signal switch $S_s$, herein shown as being a solid state device No. 4066, and the output of the switch $S_s$ is connected to a point of reference potential by the capacitor 18 across which the substitute pulse $P_S$ indicated in Figure 4f appears. As in Figure 4, the output terminal 20 is connected to the side of the capacitor 18 that is connected to reference potential, and the other output terminal 16 is at the other side of the capacitor 18. In order to prevent subsequent devices from placing an impedance in shunt with the capacitor 18 that could interfere with the operation of the circuit, the output terminal 16 is connected via a protection resistor 54 and a buffer amplifier 56 to another output terminal 16′.

The control of the switch $S_s$ as described in connection with Figure 4 is attained by the following circuit elements of Figure 4G. The differentiator 24 is comprised of a resistor 64 and a capacitor 66 connected in series in the order named between the output of the amplifier 40 and one input of a differential amplifier 58, and the other input of the amplifier 58 is connected to a point of reference potential. A resistor 68 is connected between the output of the amplifier 58 and the input that is connected to the capacitor 66. As is well understood by those skilled in the art, the amplifier circuit just described will differentiate the pace pulse P appearing at the output of the amplifier 40 so as to produce at its output a wave 70 having the first pulse $S_L$ of Figure 4b coincident with the leading edge of the pace pulse P and of the same polarity with respect to the reference potential, and a second pulse $S_T$ of Figure 4b coincident with the trailing edge of the pace pulse P and of the opposite polarity.

The output of the differentiating amplifier 58 is connected to the bipolar comparator 26 by a connection to the inverting inputs of differential amplifiers 72 and 74 contained within a chip 76, herein indicated as being No. LM-339. The outputs of the amplifiers are connected to a point of potential that is positive with respect to the reference potential via an output resistor 75. Resistors 77, 78 and 80 are connected in series in the order named between a point of voltage that is negative with respect to the reference potential and a point of voltage that is positive thereto. The values of the resistors 77, 78 and 80 are so chosen that the junction of the resistors 77 and 78, which is connected to the noninverting input of the amplifier 72, is negative with respect to the reference potential, as indicated by a dashed line 82 shown with the wave 70; and the junction of the resistors 78 and 80, which is connected to the noninverting input of the amplifier 74, is positive with respect to the reference potential, as indicated by the dashed line 84 shown with the wave 70.

The operation of the chip 76 and its associated circuit as a bipolar comparator is as follows. When the positive pulse $S_L$ at the output of the differential amplifier 58 becomes more positive than the voltage indicated by the dashed line 84, the output of the differential amplifier 72 goes negative as indicated by the negative-going output signal 55. Should the polarity of the pace pulse P be negative instead of positive, as shown, the pulse $S_L$ occurring at its leading edge will be negative. When it becomes more negative than the voltage indicted by the dashed line 82, the output of the amplifier 72 goes negative as indicated at 55. In this particular embodiment of the invention, no use is made of the pulse $S_T$ formed by the differential amplifier 58.

The functions of the switch control means 28 of Figure 4 are carried out by a chip 86, type No. 4538. Its negative-triggered input terminal $B_1$ is connected to the outputs of the amplifiers 72 and 74, and it includes within it, two monostable multivibrators corresponding to the multivibrators 30 and 32 of Figure 4. One multivibrator is controlled by external circuitry so as to be in an unstable condition for the duration of the short period indicated by the pulse $P_{30}$ of Figure 4d; and the other is controlled by external circuitry so as to be in an unstable condition for the long period indicated by the pulse $P_{32}$ of Figure 4e. The short-period multivibrator is controlled by the connection of a resistor 88 between a terminal two and a point of positive operating potential and connecting a resistor 90 and a capacitor 92 in series between the terminal two and a point of negative operating potential. The long-period multivibrator, which is actuated by the positive-going edge of the short-period multivibrator, is controlled by the connection of a resistor 94 between a terminal fourteen and the point of positive operating potential and by connecting a resistor 96 and a capacitor 98 in series between the terminal fourteen and the point of negative operating potential. Terminals eleven, thirteen and sixteen are connected to the point of positive operating potential, and terminals one, eight and fifteen are connected to the point of negative operating potential. Terminals four and six are connected together, as are terminals seven and twelve. A negative output pulse such as $P_{32}$ shown in Figure 4e appears at the connected terminals three and nine and is applied by a lead 100 to terminal thirteen of the chip 86 so as to place it in a nonconductive state and cause an output pulse such as shown in Figure 4f to appear across the output 16, 20 that is proportional to the amplitude of the pace pulse P.

Figure 4' is a modification of Figure 4 that provides an independently adjustable response to the pace pulse P by modifying the time constant of the integration circuit to which the pace pulse P is applied. Those components having the same function as in Figure 4 are designated in the same way and the waveforms of Figures 4a through 4c and Figures 4d', 4e' and 4f' appear at like-lettered points. A normally nonconductive auxiliary switch $S_A$ and a resistor 102 are connected in series between the input 10, 12 and the output 16, 20. The differentiation circuit 24 and the bipolar comparator 26 of the pace pulse sensing means 22 operate as in Figure 4 to produce the negative pulses $P_L$ and $P_T$ of Figure 4c. The pulse $P_L$ at the leading edge of a pace pulse P triggers a multivibrator 104 so as to cause it to produce the positive pulse shown in Figure 4d' that is applied so as to close the auxiliary switch $S_A$ and charge the output capacitor 18 with a portion of the pace pulse P through the resistor 102. The duration of the pulse shown in Figure 4d' and the value of the resistor 102 can be selected to produce a desired voltage across the capacitor 18. The normally closed signal switch $S_S$ is opened at the beginning of the pace pulse P and reclosed following the after-voltage by coupling a multivibrator 106 between it and the output of the bipolar comparator 26. The multivibrator 106 has an output such as indicated in Figure 4e'.

Figure 5 is a block diagram of a first embodiment of a second specie of the invention in which the substitute pulse $P_S$ applied to the output 16, 20 in place of the pace pulse P has an amplitude that is related to the product of the amplitude and duration of the pace pulse P. This is effected by charging the capacitor 18 during the entire pace pulse P rather than during just a part of it. Components corresponding to those in Figure 4 have the same designations and the waveforms of Figures 5a through 5g and the pulse $P_S$ of Figure 5h appear at identically lettered points in Figure 5.

The pace pulse sensing means 22 is the same as in Figure 4, but it has a signal switch control means 107 that differs from the signal switch control means of Figure 4. The signal switch control means 107 is comprised of a toggle multivibrator 108 that is triggered by the pulses $P_L$ and $P_T$ supplied by the comparator 26 so as to output a pulse $P_{108}$ shown in Figure 5d which is applied to a multivibrator 110 and one input of an EXCLUSIVE NOR gate 112. As shown in Figure 5e, the multivibrator 110 outputs a pulse $P_{110}$ that begins at the rising edge of the pulse $P_{108}$ and lasts until any after-voltage has an insignificant amplitude. The pulse $P_{110}$ is applied to the other input of the EXCLUSIVE NOR gate 112 so that its output remains high until the end of the pace pulse P at which point it becomes low until the end of the pulse $P_{110}$. Thus, the signal switch $S_S$ remains closed during the entire pace pulse and is opened immediately thereafter during the after-voltage so that the capacitor 18 is charged during the entire pace pulse.

Since the toggle multivibrator 108 could be triggered by a narrow noise spike so as to be in an improper state when a pace pulse P arrives, a very short pulse $P_{114}$ of about 10 microseconds (shown in Figure 5f) is developed in response to the termination of the pulse $P_{110}$ by a monostable multivibrator 114 and applied to a reset input of the toggle multivibrator 108.

Figure 5' illustrates a second embodiment of the second specie of the invention in which the amplitude of the substitute pulse $P_S$ is independently adjustable. Corresponding components are designated in the same way as in Figure 5 and the waveforms of Figures 5a through 5f and the substitute pulses $P_S'$ and $P_S''$ of Figure 5h occur at the point H.

A normally open auxiliary switch $S_A$ is connected in series with a reistor 116 between the input 10, 12 and the output 16, 20. A switch control means 118 is comprised of the toggle multivibrator 120 that is triggered by the pulses $P_L$ and $P_T$ at the output of the comparator 26 so as to produce a pulse like the pulse $P_{108}$ shown in Figure 5d that is positive during the pace pulse P. The pulse at the output of the multivibrator 120 is applied to the auxiliary switch $S_A$ so as to close it during each pace pulse, and to a multivibrator 121 so as to trigger it into an unstable state. The multivibrator 121 outputs a pulse like the pulse $P_{110}$ of Figure 5e that is inverted by an inverter 122 and applied to the signal switch $S_s$ so as to place it in a nonconducting state from the leading edge of a pace pulse P until the amplitude of any after-voltage has a negligible value. The multivibrator 123 serves the same purpose as in Figure 5. By making the value of the resistor 116 less than that of the resistor 14 of Figure 5, the amplitude of the substitute pulse can be increased from that produced by the circuit of Figure 5 as indicated at $P_S'$ of Figure 5h; and by making the value of the resistor 116 greater than that of the resistor 14 of Figure 5, the amplitude of the substitute pulse can be decreased from that produced by the circuit of Figure 5 as indicated at $P_S''$ of Figure 5h.

Figure 6 illustrates a first embodiment of a third specie of the invention in which the substitute pulse $P_S$ supplied to the output 16, 20 is generated by charging the output capacitor 18 with a constant current source in such manner that the substitute pulse $P_S$ has an amplitude relating only to the width of the pace pulse P. Those components corresponding to those in Figure 5 are designated in the same way and the waveforms of Figures 6a through 6g appear at like-lettered points. A normally nonconducting auxiliary switch $S_A$ is connected so as to place a constant current source 124 in parallel with the capacitor 18 when it is conducting. As previously explained in connection with Figure 5, the presence of a pace pulse P at the input 10, 12 causes the bipolar comparator 26 to output pulses $P_L$ and $P_T$ respectively at the leading and trailing edges of the pace pulse P. The pulse $P_L$ is applied to a toggle or bistable multivibrator 126 so as to cause its output to assume a high stable state, and the pulse $P_T$ causes the output of the multivibrator 126 to assume a low stable state so as to form the pulse $P_{126}$ of Figure 6d that lasts during the entire pace pulse P. The pulse $P_{126}$ is inverted in an inverter 128 and applied so as to make the normally nonconducting auxiliary switch $S_A$ conductive and to cause the capacitor 18 to be charged from the constant current source 124 and produce the substitute pulse $P_S$ shown in Figure 6g. The signal switch $S_s$ is controlled as follows. The leading edge of the pulse $P_{126}$ causes a monostable multivibrator 130 to output a low state pulse $P_{130}$ of Figure 6e that is applied so as to make the normally conductive signal switch $S_s$ nonconductive for a period beginning at the leading edge of the pace pulse P and ending some time after the trailing edge of the pace pulse P when the after-voltage AV has diminished. The multivibrator 114 prevents improper operation in the presence of noise, as explained in connection with Figure 5.

If it is desired that the substitute pulse $P_S$ derived at the output 16, 20 have less amplitude, the auxiliary switch $S_A$ can be made conductive for only the first portion of a pace pulse, as shown in Figure 6' in which components corresponding to those of Figure 6 are designated in the same way and the waveforms of Figures 6'a through 6'f appear at like-lettered points. In this second embodiment of the third specie of the invention, the pulse $P_L$ activates a monostable multivibrator 132 of a means 134 for controlling switches $S_s$ and $S_A$ so as to cause the multivibrator 132 to output a high state pulse $P_{132}$ of Figure 6'd. The pulse $P_{132}$ is inverted by an inverter 136 and applied so as to make the normally conductive signal switch $S_s$ nonconductive during a period beginning at the leading edge of the pace pulse P and ending when the after-voltage AV has diminished sufficiently.

The pulse $P_{132}$ is also applied so that its leading edge triggers a monostable multivibrator 138 that stays in its unstable condition for less than the duration of a pace pulse P and outputs a pulse $P_{138}$ of Figure 6'e. This pulse is applied via an inverter 140 so as to make the normally nonconductive auxiliary switch $S_A$ conductive, thereby causing the capacitor 18 to be charged from the current source 124 and produce the substitute pulse $P_S$ shown in Figure 6'f that lasts until the signal switch $S_s$ is made conductive again at the end of the pulse $P_{132}$.

Figure 7 shows an EKG system having six leads $L_1$ through $L_6$, one of which is connected to a coupling circuit 150 of this invention, and signal switches $S_{s1}$ through $S_{s6}$ that are respectively connected in series with each lead and operated by the signal switch control means of the coupling circuit 150 so as to prevent the after-voltages that may follow the pace pulses on of the leads $L_1$ through $L_6$ from reaching a sampling display 152.

**Claims**

1. Apparatus for processing EKG signals having pace pulses therein, comprising:
— an input to which EKG signals and accompanying pace pulses may be applied,
— an output at which the processed signals appear,
— pace pulse sensing means (22) coupled to the input (10, 12) for sensing the presence of a pace pulse at the input and comprising a differentiator (24) coupled to the input (10, 12) and a bipolar comparator (26) coupled to the differentiator,

— a normally closed switch ($S_s$) for interrupting the electrical connection of the input (10, 12) and the output (16, 20),

— switch control means (28) coupled to the pace pulse sensing means for opening the switch for a period beginning during the appearance of the pace pulse and for closing the switch at a predetermined time thereafter, and

— pulse generating means for deriving a substitute pulse from the pace pulse and for inserting said substitute pulse into the sequence of EKG signals appearing at the output at the position of the original pace pulse,

characterized in that the pulse generating means comprises an integrating circuit (14, 18) for integrating a portion of the pace pulse, said integrating circuit containing a resistor (14) connected in series to the switch ($S_s$) and a capacitor (18) coupled across the output (16, 20), the time constant of said resistor and said capacitor being such as to integrate a pace pulse, that for generating a substitute pulse the amplitude of which is proportional to the amplitude of the pace pulse and the amplitude of which is adjustable independently of the amplitudes of the other EKG signals, a normally open auxiliary switch ($S_A$) and a resistor (102, 116) connected in series thereto are provided between the input (10, 12) and the junction of the resistor (14) and the capacitor (18) of the integrating circuit, and that auxiliary switch control means is provided for closing the auxiliary switch at the occurrence of the leading edge of the pace pulse and for opening the auxiliary switch a predetermined time thereafter before the occurrence of the trailing edge of the pace pulse.

2. Apparatus according to claim 1, characterized in that for generating a substitute pulse the amplitude of which is proportional to the product of the amplitude and the width of the pace pulse the switch control means is designed for opening the switch ($S_s$) at the occurrence of the leading edge of the pace pulse and for closing the switch a predetermined time thereafter after the occurrence of the trailing edge of the pace pulse.

3. Apparatus for processing EKG signals having pace pulses therein, comprising:

— an input to which EKG signals and accompanying pace pulses may be applied,

— an output at which the processed signals appear,

— pace pulse sensing msans (22) coupled to the input (10, 12) for sensing the presence of a pace pulse at the input and comprising a differentiator (24) coupled to the input (10, 12) and a bipolar comparator (26) coupled to the differentiator,

— a normally closed switch ($S_s$) for interrupting the electrical connection of the input (10, 12) and the output (16, 20),

— switch control means (107) coupled to the pace pulse sensing means for opening the switch for a period beginning during the appearance of a pace pulse and for closing the switch at a predetermined time thereafter, and

— pulse generating means for deriving a substitute pulse from the pace pulse and for inserting said substitute pulse into the sequence of EKG signals appearing at the output at the position of the original pace pulse,

characterized in that for generating a substitute pulse the amplitude of which is proportional to the product of the amplitude and the width of the pace pulse the switch control means is designed for opening the switch ($S_s$) for a predetermined time beginning at the occurence of the trailing edge of the pace pulse, thus integrating the pace pulse.

4. Apparatus for processing EKG signals having pace pulses therein, comprising:

— an input to which EKG signals and accompanying pace pulses may be applied,

— an output at which the processed signals appear,

— pace pulse sensing means (22) coupled to the input (10, 12) for sensing the presence of a pace pulse at the input and comprising a differentiator (24) coupled to the input (10, 12) and a bipolar comparator (26) coupled to the differentiator,

— a normally closed switch ($S_s$) for interrupting the electrical connection of the input (10, 12) and the output (16, 20),

— switch control means (107) coupled to the pace pulse sensing means for opening the switch for a period beginning during the appearance of a pace pulse and for closing the switch at a predetermined time thereafter, and

— pulse generating means for deriving a substitute pulse from the pace pulse and for inserting said substitute pulse into the sequence of EKG signals appearing at the output at the position of the original pace pulse,

characterized in that for generating a substitute pulse the amplitude of which is proportional to the width of the pace pulse a current source (124) coupled to the integrating means and a normally open auxiliary switch ($S_A$) between the current source and the integrating circuit means are provided, said auxiliary switch being operated by an auxiliary switch control means,

that the auxiliary switch control means is designed for closing the auxiliary switch at the occurrence of the leading edge of the pace pulse and for opening the auxiliary switch at the occurrence of the trailing edge of the pace pulse, and that the switch control means is designed for opening the switch ($S_s$) at the occurrence of the leading edge of the pace pulse and for closing the switch after the occurrence of the trailing edge of the pace pulse.

**Patentansprüche**

1. Vorrichtung zum Verarbeiten von EKG-Signalen, die Schrittmacherimpulse enthalten, wobei die Vorrichtung umfaßt:

— einen Eingangsanschluß, an den EKG-Signale und begleitende Schrittmacherimpulse angelegt werden können,

— einen Ausgangsanschluß, an dem die verarbeiteten Signale erscheinen,
— Schrittmacherimpuls-Erfassungsmittel (22), die zum Erfassen des Auftretens von Schrittmacherimpulsen an dem Eingangsanschluß (10, 12) mit dem Eingangsanschluß verbunden sind und einen Differentiator (24), der mit dem Eingangsanschluß (10, 12) verbunden ist, und einen bipolaren Komparator (26) umfassen, der mit dem Differentiator verbunden ist,
— einen normalerweise geschlossenen Schalter ($S_s$) zum Unterbrechen der elektrischen Verbindung des Eingangsanschlusses (10, 12) und des Ausgangsanschlusses (16, 20),
— Schalterüberwachungsmittel (28), die mit den Schrittmacherimpuls-Erfassungsmitteln verbunden sind, zum Öffnen des Schalters für eine Zeitspanne, welche während des Auftretens eines Schrittmacherimpulses beginnt, und zum Schließen des Schalters zu einem vorbestimmten Zeitpunkt danach, und
— Impulserzeugungsmittel zum Ableiten eines Ersatzimpulses von dem Schrittmacherimpuls und zum Einfügen des Ersatzimpulses in die Folge EKG-Signale, welche an dem Ausgangsanschluß erscheinen, und zwar an der Stelle des ursprünglichen Schrittmacherimpulses,
dadurch gekennzeichnet, daß die Impulserzeugungsmittel eine Integrierschaltung (14, 18) zum Integrieren eines Teiles des Schrittmacherimpulses umfassen, wobei die Integrierschaltung einen Widerstand (14), der in Serie mit dem Schalter ($S_s$) verschaltet ist, und einen Kondensator (18) umfaßt, der über dem Ausgangsanschluß (16, 20) liegt, wobei die Zeitkonstante des Widerstandes und des Kondensators so ist, daß ein Schrittmacherimpuls integriert wird, daß zum Erzeugen eines Ersatzimpulses, dessen Amplitude proportional zu der Amplitude des Schrittmacherimpulses ist und dessen Amplitude unabhängig von den Amplituden der anderen EKG-Signale einstellbar ist, ein normalerweise offener Hilfsschalter ($S_A$) und eine Widerstand (102, 116) dazu in Serie zwischen dem Eingangsanschluß (10, 12) und der Verbindung des Widerstandes (14) und des Kondensators (18) der Integrierschaltung vorgesehen sind, und daß Hilfsschalter-Überwachungsmittel vorgesehen sind zum Schließen des Hilfsschalters bei Auftreten der führenden Flanke des Schrittmacherimpulses und zum Öffnen des Hilfsschalters eine vorbestimmte Zeitspanne danach, und zwar vor dem Auftreten der rückwärtigen Flanke des Schrittmacherimpulses.

2. Vorrichtung nach Ankqruch 1, dadurch gekennzeichnet, daß zum Erzeugen eines Ersatzimpulses, dessen Amplitude proportional zum dem Produkt der Amplitude und der Breite des Schrittmacherimpulses ist, die Schalterüberwachungsmittel so ausgelegt sind, daß sie den Schalter ($S_s$) bei Auftreten der führenden Flanke des Schrittmacherimpulses öffnen und den Schalter eine vorbestimmte Zeitspanne danach schließen, und zwar nach dem Auftreten der rückwärtigen Flanke des Schrittmacherimpulses.

3. Vorrichtung zum Verarbeiten von EKG-Signalen, die Schrittmacherimpulse enthalten, wobei die Vorrichtung umfaßt:
— einen Eingangsanschluß, an den EKG-Signale und begleitende Schrittmacherimpulse angelegt werden können,
— einen Ausgangsanschluß, an dem die verarbeiteten Signale erscheinen,
— Schrittmacherimpuls-Erfassungsmittel (22), die zu Erfassen des Auftretens von Schrittmacherimpulsen an dem Eingangsanschluß (10, 12) mit dem Eingangsanschluß verbunden sind une einen Differentiator (24), der mit dem Eingangsanschluß (10, 12) verbunden ist, und einen bipolaren Komparator (26) umfassen, der mit dem Differentiator verbunden ist,
— einen normalerweise geschlossenen Schalter ($S_s$) zum Unterbrechen der elektrischen Verbindung des Eingangsanschlusses (10, 12) und des Ausgangsanschlusses (16, 20),
— Schalterüberwachungsmittel (107), die mit den Schrittmacherimpuls-Erfassungsmitteln verbunden sind, zum Öffnen des Schalters für eine Zeitspanne, welche während des Auftretens eiens Schrittmacherimpulses beginnt, und zum Schließen des Schalters zu einem vorbestimmten Zeitpunkt danach, und
— Impulserzeugungsmittel zum Ableiten eines Ersatzimpulses von dem Schrittmacherimpuls und zum Einfügen des Ersatzimpulses in die Folge EKG-Signale, welche andem Ausgangsanschluß erscheinen, und zwar an der Stelle des ursprünglichen Schrittmacherimpulses,
dadurch gekennzeichnet, daß zum Erzeugen eines Ersatzimpulses, dessen Amplitude proportional zu dem Produkt der Amplitude und der Breite des Schrittmacherimpulses ist, die Schalterüberwachungsmittel so ausgelegt sind, daß sie den Schalter ($S_s$) für eine vorbestimmte Zeitspanne öffnen, welche bei Auftreten der rückwärtigen Flanke des Schrittmacherimpulses beginnt, wodurch der Schrittmacherimpuls integriert wird.

4. Vorrichtung zum Verarbeiten von EKG-Signalen, die Schrittmacherimpulse enthalten, wobei die Vorrichtung umfaßt:
— einen Eingangsanschluß, an den EKG-Signale und begleitende Schrittmacherimpulse angelegt werden können,
— einen Ausgangsanschluß, an dem die verarbeiteten Signale erscheinen,
— Schrittmacherimpuls-Erfassungsmittel (22), die zum Erfassen des Auftretens von Schrittmacherimpulsen an dem Eingangsanschluß (10, 12) mit dem Eingangsanschluß verbunden sind und einen Differentiator (24), der mit dem Eingangsanschluß (10, 12) verbunden ist, und einen bipolaren Komparator (26) umfassen, der mit dem Differentiator verbunden ist,
— einen normalerweise geschlossenen Schalter ($S_s$) zum Unterbrechen der elektrischen Verbindung des Eingangsanschlusses (10, 12) und des Ausgangsanschlusses (16, 20),
— Schalterüberwachungsmittel (107), die mit den Schrittmacherimpuls-Erfassungsmitteln verbunden sind, zum Öffnen des Schalters für eine Zeitspanne, welche während des Auftretens eines Schrittmacherimpulses beginnt, und zum Schließen des Schalters zu einem vorbestimmten Zeitpunkt danach, und

— Impulserzeugungsmittel zum Ableiten eines Ersatzimpulses von dem Schrittmacherimpuls und zum Einfügen des Ersatzimpulses in die Folge EKG-Signale, welche an dem Ausgangsanschluß erscheinen, und zwar an der Stelle des ursprünglichen Schrittmacherimpulses,

dadurch gekennzeichnet, daß zum Erzeugen eines Ersatzimpulses, dessen Amplitude proportional zu der Breite des Schrittmacherimpulses ist, eine Stromquelle (124), die mit der Integrierschaltung verbunden ist, und ein normalerweise offener Hilfsschalter (S$_A$) zwischen der Stromquelle und der Integrierschaltung vorgesehen sind, wobei der Hilfsschalter von Hilfsschalter-Überwachungsmitteln gesteuert wird, daß die Hilfsschalter-Überwachungsmittel dazu ausgelegt sind, den Hilfsschalter bei Auftreten der führenden Flanke des Schrittmacherimpulses zu schließen und den Hilfsschalter bei Auftreten der rückwärtigen Flanke des Schrittmacherimpulses zu öffnen, und daß die Schalterüberwachungsmittel dazu ausgelegt sind, den Schalter (S$_s$) bei Auftreten der führenden Flanke des Schrittmacherimpulses zu öffnen und den Schalter nach dem Auftreten der rückwärtigen Flanke des Schrittmacherimpulses zu schließen.

**Revendications**

1. Appareil pour traiter des signaux ECG contenant des impulsions de stimulation, comprenant:

— une entrée à laquelle des signaux ECG et des impulsions de stimulation d'accompagnement peuvent être appliqués,

— une sortie à laquelle apparaissent les signaux traités,

— un moyen (22) de détection d'impulsions de stimulation, relié à l'entrée (10, 12) pour détecter la présence d'une impulsion de stimulation à l'entrée, et comprenant un différentiateur (24) relié à l'entrée (10, 12) et un comparateur bipolaire (26) relié au différentiateur,

— un interrupteur normalement fermé (S$_s$) pour interrompre la liaison électrique entre l'entrée (10, 12) et la sortie (16, 20),

— un moyen (28) de commande d'interrupteur, relié au moyen de détection d'impulsions de stimulation pour ouvrir l'interrupteur pendant une période commençant pendant l'apparation d'une impulsion de stimulation et pour fermer l'interrupteur à un instant ultérieur prédéterminé, et

— un moyen générateur d'impulsions pour dériver une impulsion de substitution de l'impulsion de stimulation et pour introduire ladite impulsion de substitution dans la séquence de signaux ECG apparaissant à la sortie dans la position de l'impulsion de stimulation d'origine,

caractérisé en ce que le moyen générateur d'impulsions comprend un circuit intégrateur (14, 18) pour intégrer une partie de l'impulsion de stimulation, ledit circuit intégrateur contenant une résistance (14) reliée en série à l'interrupteur

(S$_s$) et un condensateur (18) monté entre les bornes de la sortie (16, 20), la constante de temps de ladite résistance et dudit condensateur permettant l'intégration d'une impulsion de stimulation, en ce que pour la génération d'une impulsion de substitution dont l'amplitude est proportionnelle à l'amplitude de l'impulsion de stimulation et dont l'amplitude est réglable indépendamment des amplitudes des autres signaux ECG, il est prévu un interrupteur auxiliaire (S$_A$) normalement ouvert et une résistance (116) reliée en série avec celui-ci entre l'entrée (10, 12) et la jonction de la résistance (14) et du condensateur (18) du circuit d'intégration, et en ce qu'il est prévu un moyen de commande d'interrupteur auxiliaire pour fermer l'interrupteur auxiliaire lors de l'apparition du front avant de l'impulsion de stimulation et pour ouvrir l'interrupteur auxiliaire à un instant ultérieur prédéterminé et avant l'apparition du front arrière de l'impulsion de stimulation.

2. Appareil selon la revendication 1, caractérisé en ce que, pour la génération d'une impulsion de substitution dont l'amplitude est proportionnelle au produit de l'amplitude et de la largeur de l'impulsion de stimulation, le moyen de commande d'interrupteur est conçu pour ouvrir l'interrupteur (S$_s$) lors de l'apparition du front avant de l'impulsion de stimulation et pour fermer l'interrupteur à un instant ultérieur prédéterminé après l'apparition du front arrière de l'impulsion de stimulation.

3. Appareil pour traiter des signaux ECG contenant des impulsions de stimulation, comprenant:

— une entrée à laquelle des signaux ECG et des impulsions de stimulation d'accompagnement peuvent être appliqués,

— une sortie à laquelle apparaissent les signaux traités,

— un moyen (107) de commande d'interrupteur, relié au moyen de détection d'impulsions de stimulation pour ouvrir l'interrupteur pendant une période commençant pendant l'apparation d'une impulsion de stimulation et pour fermer l'interrupteur à un instant ultérieur prédéterminé, et

— un moyen générateur d'impulsions pour dériver une impulsion de substitution de l'impulsion de stimulation et pour introduire ladite impulsion de substitution dans la séquence de signaux ECG apparaissant à la sortie dans la position de l'impulsion de stimulation d'origine,

caractérisé en ce que, pour la génération d'une impulsion de substitution dont l'amplitude est proportionnelle au produit de l'amplitude et de la largeur de l'impulsion de stimulation, le moyen de commande d'interrupteur est conçu pour ouvrir l'interrupteur (S$_s$) pendant un temps prédéterminé commençant lors de l'apparition du front arrière de l'impulsion de stimulation, en intégrant ainsi l'impulsion de stimulation.

4. Appareil pour traiter des signaux ECG contenant des impulsions de stimulation, comprenant:

— une entrée à laquelle des signaux ECG et des impulsions de stimulation d'accompagnement peuvent être appliqués,

— une sortie à laquelle apparaissent les signaux traités,

— un moyen (22) de détection d'impulsions de stimulation, relié à l'entrée (10, 12) pour détecter la présence d'une impulsion de stimulation à l'entrée, et comprenant un différentiateur (24) relié à l'entrée (10, 12) et un comparateur bipolaire (26) relié au différentiateur,

— un interrupteur normalement fermé (S$_s$) pour interrompre la liaison électrique entre l'entrée (10, 12) et la sortie (16, 20),

— un moyen (107) de commande d'interrupteur, relié au moyen de détection d'impulsions de stimulation pour ouvrir l'interrupteur pendant une période commençant pendant l'apparition d'une impulsion de stimulation et pour fermer l'interrupteur à un instant ultérieur prédéterminé, et

— un moyen générateur d'impulsions pour dériver une impulsion de substitution de l'impulsion de stimulation et pour introduire ladite impulsion de substitution dans la séquence de signaux ECGT apparaissant à la sortie dans la position de l'impulsion de stimulation d'origine, caractérisé en ce que, pour la génération d'une impulsion de substitution dont l'amplitude est proportionnelle à la largeur de l'impulsion de stimulation, il est prévu une source de courant (124) couplée avec le moyen d'intégration et un interrupteur auxiliaire (S$_A$) normalement ouvert, entre la source de courant et le circuit d'intégration, ledit interrupteur auxiliaire étant activé par un moyen de commande d'interrupteur auxiliaire, en ce que le moyen de commande d'interrupteur auxiliaire est conçu pour fermer l'interrupteur auxiliaire lors de l'apparition du front avant de l'impulsion de stimulation et pour ouvrir l'interrupteur auxiliaire lors de l'apparition du front arrière de l'impulsion de stimulation, et en ce que le moyen de commande d'interrupteur est conçu pour ouvrir l'interrupteur (S$_s$) lors de l'apparition du front avant de l'impulsion de stimulation et pour fermer l'interrupteur après l'apparition du front arrière de l'impulsion de stimulation.

Fig 1

Fig 4

Fig 4'

FIG 4a, FIG 4b, FIG 4c, FIG 4d, FIG 4e, FIG 4f, FIG 4d', FIG 4e', FIG 4f', Fig. 2a, Fig. 2b, Fig. 3a, Fig. 3b

2

Fig 4G

3

$\overline{\mp}ig$ 5

$\overline{\mp}ig$ 5'

EP 0 142 161 B1

FIG
5 a

FIG
6a

FIG
6' a

F IG
5 b

FIG
6 b

FIG
6' b

FIG
5 c

FIG
6c

FIG
6'c

P108 FIG
5d

P124 FIG
6 d

P134 FIG
6'd

P110 FIG
5e

P130 FIG
6e

P138 FIG
6'e

P114 FIG
5f

FIG
6f

PS FIG
6'f

FIG
5g

PS FIG
6g

FIG
6'g

PS' FIG
PS" PS 5h

Fig 6

Fig 6'

Fig 7